# EUROPEAN PATENT APPLICATION

(11) **EP 4 083 197 A1**
(43) Date of publication of application: **02.11.2022**
(21) Application number: 21182464.4
(22) Date of filing: 29.06.2021
(51) Int. Cl.: C12N 9/00, C12N 15/77, C12P 13/08, C12R 1/15

(54) **CORYNEBACTERIUM GLUTAMICUM MUTANT STRAIN HAVING ENHANCED L-LYSINE PRODUCTIVITY AND METHOD OF PRODUCING L-LYSINE USING THE SAME**

(30) Priority: 30.04.2021 KR 20210056581; 25.05.2021 KR 20210066965
(71) Applicant: Daesang Corporation, Dongdaemun-gu, Seoul 02586 (KR)
(72) Inventor: CHOI, Min Jin, 17384 Gyeonggi-do (KR); KIM, Bong Ki, 07280 Seoul (KR); KIM, Ha Eun, 44932 Ulsan (KR); PARK, Seok Hyun, 12258 Gyeonggi-do (KR); PARK, Joon Hyun, 13523 Gyeonggi-do (KR)
(74) Representative: Nony

(57) **Abstract**

The present disclosure relates to a *Corynebacterium glutamicum* mutant strain having enhanced L-lysine productivity and a method of producing L-lysine using the same. The mutant strain may produce L-lysine in an improved yield due to increased or enhanced expression of the gene encoding pyruvate carboxylase, compared to a parent strain thereof.

## Description

### BACKGROUND

### 1. Technical Field

The present disclosure relates to a *Corynebacterium glutamicum* mutant strain having enhanced L-lysine productivity and a method of producing L-lysine using the same.

### 2. Related Art

L-lysine is an essential amino acid that is not synthesized in the human or animal body. L-lysine needs to be supplied externally and is generally produced by fermentation using microorganisms such as bacteria or yeast. L-lysine production may be performed using naturally occurring wild-type strains or mutant strains obtained by modifying the wild-type strains to have enhanced L-lysine productivity. In recent years, in order to improve the production efficiency of L-lysine, various recombinant strains or mutant strains having excellent L-lysine productivity and methods of producing L-lysine using the same have been developed by applying gene recombination technology to microorganisms such as *Escherichia coli* and *Corynebacterium,* which are widely used for the production of L-amino acids and other useful substances.

According to Korean Patent Nos. 10-0838038 and 10-2139806, L-lysine productivity may be enhanced by increasing the expression of genes encoding proteins, including L-lysine production-related enzymes, through modification of the nucleotide sequences of the genes or the amino acid sequences of the proteins, or by removing unnecessary genes. In addition, Korean Patent Application Publication No. 10-2020-0026881 discloses a method by which the existing promoter of a gene encoding an enzyme involved in L-lysine production is changed to a promoter having strong activity in order to increase the expression of the gene.

As described above, various methods for increasing L-lysine productivity have been developed, but there are dozens of types of proteins such as enzymes, transcription factors, and transport proteins, which are directly or indirectly involved in L-lysine production. Hence, extensive studies still need to be conducted on whether or not changes in the activities of these proteins lead to an increase in L-lysine productivity.

### [Prior Art Documents]

### [Patent Documents]

Korean Patent No. 10-0838038
Korean Patent No. 10-2139806
Korean Patent Application Publication No. 10-2020-0026881

### SUMMARY

An object of the present disclosure is to provide a *Corynebacterium glutamicum* mutant strain having enhanced L-lysine productivity.

Another object of the present disclosure is to provide a method of producing L-lysine using the mutant strain.

The present inventors have conducted studies to develop a novel mutant strain having enhanced L-lysine productivity using a *Corynebacterium glutamicum* strain, and as a result, have found that, when the nucleotide sequence at specific positions in the promoter of the pyc gene encoding pyruvate carboxylase involved in the supply of the lysine precursor oxaloacetate in the L-lysine biosynthesis pathway is substituted, the production of L-lysine increases while the production of pyruvate-derived by-products decreases, thereby completing the present disclosure.

One aspect of the present disclosure provides a *Corynebacterium glutamicum* mutant strain having enhanced L-lysine productivity due to enhanced activity of pyruvate carboxylase.

As used herein, the term "pyruvate carboxylase" refers to an enzyme that catalyzes a reaction that produces the lysine precursor oxaloacetate (OAA) by inducing the carboxylation of pyruvate in the L-lysine biosynthesis pathway.

According to one embodiment of the present disclosure, the pyruvate carboxylase may be derived from a *Corynebacterium* sp. strain. Specifically, the *Corynebacterium* sp. strain may be, but is not limited to, *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens.*

As used herein, the term "enhanced activity" means that the expression of a gene encoding a protein such as a target enzyme, transcription factor or transport protein is newly introduced or increased, so that the expression level of the gene is increased compared to that in the wild-type strain or the strain before modification. The term "enhanced activity" also includes: a case in which the activity of the protein itself is increased compared to the activity of the protein of the parent microorganism by substitution, insertion, deletion, or a combination thereof, of one or more of the nucleotides encoding the gene; a case in which the overall enzyme activity in the cell is higher than that in the wild-type strain or the strain before modification due to increased expression or translation of the gene encoding the protein; and a combination thereof.

According to one embodiment of the present disclosure, the enhanced activity of pyruvate carboxylase may be achieved by site-directed mutagenesis in the promoter of the gene encoding pyruvate carboxylase.

According to one embodiment of the present disclosure, the promoter of the gene encoding pyruvate carboxylase may be represented by the nucleotide sequence of SEQ ID NO: 1.

As used herein, the term "promoter" refers to a specific DNA region that regulates transcription of a gene, including a binding site for RNA polymerase that initiates mRNA transcription of a target gene. In general, the promoter is located upstream of the transcription start site. In prokaryotes, the promoter is defined as a region near the transcription start site to which RNA polymerase binds, and generally consists of two short sequences at positions -10 and -35 upstream from the transcription start site. In the present disclosure, mutation in the promoter means modifying the promoter to have a higher activity than a wild-type promoter, and the expression of the gene located downstream of the transcription start site may be increased by inducing mutation in the promoter region located upstream of the transcription start site.

According to one embodiment of the present disclosure, the enhanced activity of pyruvate carboxylase may be achieved by substitution of at least one nucleotide in the region located between positions -90 and -30 (hereinafter referred to as the -90 to -30 region) upstream from the transcription start site in the promoter sequence of the gene encoding pyruvate carboxylase.

More specifically, in the present disclosure, mutation in the promoter may be a substitution of at least one nucleotide in the -90 to -30 region, preferably a substitution of 1, 2, 3, 4, 5, 6, 7, 8, 9 or 10 contiguous, or non-contiguous nucleotides in the -90 to -30 region, the -80 to -40 region, the -75 to -45 region, the -55 to -40 region, or the -75 to -60 region.

According to one example of the present disclosure, a *Corynebacterium glutamicum* mutant strain having a new promoter sequence of the pyc gene was obtained by substitution of tgtggtatgatgg for the nucleotide sequence ggggttacgatac of the -73 to -61 region and substitution of acagctgctactgt for the nucleotide sequence gtgactgctatcac of the -51 to -38 region in the promoter sequence of the pyruvate carboxylase-encoding pyc gene of the *Corynebacterium glutamicum* strain. This *Corynebacterium glutamicum* mutant strain may comprise a mutated promoter of the pyc gene, which is represented by the nucleotide sequence of SEQ ID NO: 2.

In addition, according to one example of the present disclosure, a *Corynebacterium glutamicum* mutant strain having a new promoter sequence of the pyc gene was obtained by substitution of tgttgtatgattg for the nucleotide sequence ggggttacgatac of the -73 to -61 region and substitution of actgctgctactac for the nucleotide sequence gtgactgctatcac of the -51 to -38 region in the promoter sequence of the pyruvate carboxylase-encoding pyc gene of the *Corynebacterium glutamicum* strain. This *Corynebacterium glutamicum* mutant strain may comprise a mutated promoter of the pyc gene, which is represented by the nucleotide sequence of SEQ ID NO: 3.

As used herein, the term "enhanced productivity" means that L-lysine productivity of the mutant strain is increased compared to that of the parent strain. The parent strain refers to a wild-type strain or mutant strain to be mutated, and includes a strain that is to be mutated directly or to be transformed with a recombinant vector or the like. In the present disclosure, the parent strain may be a wild-type *Corynebacterium glutamicum* strain or a strain mutated from the wild-type strain.

According to one embodiment of the present disclosure, the parent strain may be a mutant strain having mutations in the sequences of genes (e.g., lysC, zwf and hom genes) that are involved in lysine production. Specifically, the parent strain may be a *Corynebacterium glutamicum* strain (hereinafter referred to as *'Corynebacterium glutamicum* DS1 strain') deposited with the Korean Culture Center of Microorganisms on April 2, 2021 under accession number KCCM12969P.

The *Corynebacterium glutamicum* mutant strain having enhanced L-lysine productivity according to the present disclosure may comprise the above-described mutated promoter sequence of the gene encoding pyruvate carboxylase.

According to one embodiment of the present disclosure, the mutant strain may comprise, as the promoter sequence of the pyruvate carboxylase-encoding gene, any one of the nucleotide sequences of SEQ ID NOs: 2 and 3.

According to one example, since the mutant strain contains mutations in the promoter of the pyc gene encoding pyruvate carboxylase, it may exhibit increased L-lysine productivity compared to the parent strain. In particular, the mutant strain may show an increase in L-lysine production of 3% or more, specifically 3 to 40%, more specifically 4 to 30%, compared to the parent strain, and thus produce 65 to 75 g, preferably 65 to 70 g of L-lysine, per liter of the strain culture medium.

The *Corynebacterium glutamicum* mutant strain according to one embodiment of the present disclosure may be obtained through a recombinant vector containing a variant resulting from substitution of a portion of the promoter sequence of the pyruvate carboxylase-encoding gene in the parent strain.

As used herein, the term "portion of the promoter sequence" means not all of the nucleotide sequence or polynucleotide sequence of the promoter, and may be, but is not limited to, 1 to 300, preferably 1 to 100, more preferably 1 to 50 nucleotides.

As used herein, the term "variant" refers to a promoter variant resulting from substitution of at least one nucleotide in the -90 to -30 region in the promoter sequence of the pyruvate carboxylase-encoding gene that is involved in the biosynthesis of L-lysine.

According to one embodiment of the present disclosure, the variant having a substitution of tgtggtatgatgg for the nucleotide sequence of the -73 to -61 region and acagctgctactgt for the nucleotide sequence of the -51 to -38 region in the promoter sequence of the pyruvate carboxylase-encoding gene may have the nucleotide sequence of SEQ ID NO: 2, and the variant having a substitution of tgttgtatgattg for the nucleotide sequence of the -73 to -61 region and actgctgctactac for the nucleotide sequence of the -51 to -38 region in the promoter sequence of the pyruvate carboxylase-encoding gene may have the nucleotide sequence of SEQ ID NO: 3.

As used herein, the term "vector" refers to an expression vector capable of expressing a protein of interest in a suitable host cell, and means a gene construct that contains essential control elements operably linked so that an inserted gene is expressed. As used herein, the term "operably linked" means that a gene to be expressed and the regulatory sequence thereof are functionally linked to each other in a manner enabling gene expression. The term "regulatory elements" includes a promoter for initiating transcription, any operator sequence for controlling transcription, a sequence encoding suitable mRNA ribosome binding sites, and a sequence for controlling termination of transcription and translation. Examples of this vector include, but are not limited to, plasmid vectors, cosmid vectors, bacteriophage vectors, and viral vectors.

The "recombinant vector" that is used in the present disclosure may be transformed into a suitable host cell, and then may replicate regardless of the genome of the host cell or may be integrated into the genome itself. In this case, the "suitable host cell" may contain a replication origin, which is a particular nucleotide sequence which enables the vector to replicate in the suitable host cell and from which replication starts.

The transformation may be performed using a suitable vector introduction technique selected depending on the host cell, so that the targeted gene may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or a combination thereof. For the transformed gene, it does not matter whether the gene is inserted into the chromosome of the host cell or located outside of the chromosome, as long as the gene may be expressed in the host cell.

The host cell may include a cell transfected, transformed, or infected with the recombinant vector or polynucleotide of the present disclosure *in vivo* or *in vitro.* The host cell containing the recombinant vector of the present disclosure may be a recombinant host cell, a recombinant cell, or a recombinant microorganism.

In addition, the recombinant vector according to the present disclosure may contain a selection marker. The selection marker may be used to select a transformant (host cell) obtained by transformation with the vector. Since only cells expressing the selection marker may survive in the medium treated with the selection marker, the selection marker may select the transformed cells. Representative examples of the selection marker include, but are not limited to, kanamycin, streptomycin, and chloramphenicol.

Genes inserted into the recombinant vector for transformation according to the present disclosure may be substituted into a host cell such as a *Corynebacterium* sp. microorganism by homologous recombination crossover.

According to one embodiment of the present disclosure, the host cell may be a *Corynebacterium* sp. strain, for example, a *Corynebacterium glutamicum* strain.

Another aspect of the present disclosure provides a method for producing L-lysine, the method including steps of: a) culturing the *Corynebacterium glutamicum* mutant strain in a medium; and b) recovering L-lysine from the mutant strain or the medium in which the mutant strain has been cultured.

The culturing may be performed using a suitable medium and culture conditions known in the art, and any person skilled in the art may easily adjust and use the medium and the culture conditions. Specifically, the medium may be a liquid medium, but is not limited thereto. Examples of the culturing method include, but are not limited to, batch culture, continuous culture, fed-batch culture, or a combination thereof.

According to one embodiment of the present disclosure, the medium should meet the requirements of a specific strain in a proper manner, and may be appropriately modified by a person skilled in the art. For the culture medium for a *Corynebacterium* sp. strain, reference may be made to, but is not limited to, a known document (Manual of Methods for General Bacteriology, American Society for Bacteriology, Washington D.C., USA, 1981).

According to one embodiment of the present disclosure, the medium may contain various carbon sources, nitrogen sources, and trace element components. Examples of the carbon sources that may be used include: saccharides and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol, and organic acids such as acetic acid. These substances may be used individually or as a mixture, but are not limited thereto. Examples of the nitrogen sources that may be used include compounds containing organic nitrogen such as peptone, yeast extract, meat extract, malt extract, corn steep liquor, soybean meal, and urea, or inorganic compounds such as ammonium sulfate, ammonium chloride, ammonium phosphate, ammonium carbonate, and ammonium nitrate. The nitrogen sources may also be used individually or as a mixture, but are not limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be added to the culture medium. The medium or individual components may be added to the culture medium batchwise or in a continuous manner by a suitable method during culturing, but are not limited thereto.

According to one embodiment of the present disclosure, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present disclosure, in the step of recovering L-lysine from the cultured mutant strain or the medium in which the mutant strain has been cultured, the produced L-lysine may be collected or recovered from the medium using a suitable method known in the art depending on the culture method. Examples of the method include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion).

According to one embodiment of the present disclosure, the step of recovering L-lysine may be performed by centrifuging the culture medium at a low speed to remove biomass and separating the obtained supernatant through ionexchange chromatography.

According to one embodiment of the present disclosure, the step of recovering L-lysine may include a process of purifying L-lysine.

### BRIEF DESCRIPTION OF THE DRAWINGS

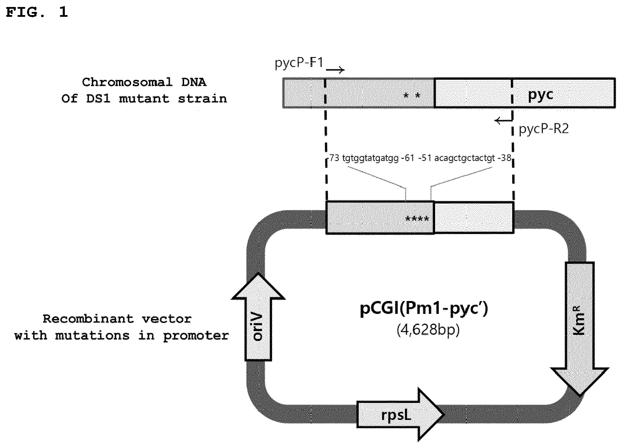

FIG. 1 shows the structure of a pCGI(Pm1-pyc') vector containing a promoter obtained by substitution of tgtggtatgatgg for the nucleotide sequence of the -73 to -61 region and substitution of acagctgctactgt for the nucleotide sequence of the -51 to -38 region in the promoter sequence of the pyruvate carboxylase-encoding gene according to one example of the present disclosure.

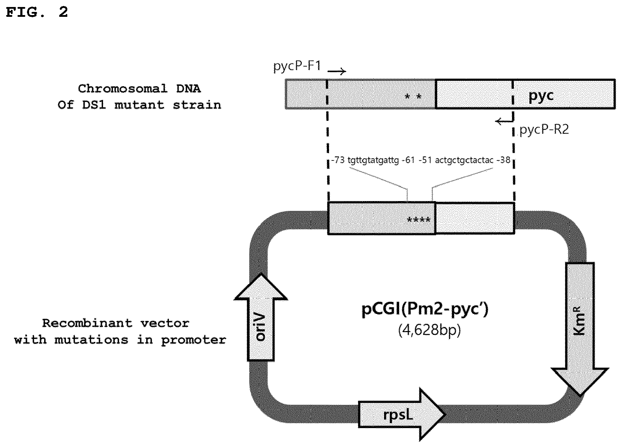

FIG. 2 shows the structure of a pCGI(Pm2-pyc') vector containing a promoter obtained by substitution of tgttgtatgattg for the nucleotide sequence of the -73 to -61 region and substitution of actgctgctactac for the nucleotide sequence of the -51 to -38 region in the promoter sequence of the pyruvate carboxylase-encoding gene according to one example of the present disclosure.

### DETAILED DESCRIPTION

Hereinafter, the present disclosure will be described in more detail. However, this description is provided by way of example only to aid the understanding of the present disclosure, and the scope of the present disclosure is not limited by this illustrative description.

### Example 1. Construction of Corynebacterium glutamicum Mutant Strain

To construct a *Corynebacterium glutamicum* mutant strain having enhanced activity of pyruvate carboxylase, random mutagenesis was performed using a *Corynebacterium glutamicum* DS1 strain.

### 1-1. Mutagenesis

The *Corynebacterium glutamicum* DS1 strain was inoculated into a flask containing 50 ml of a seed culture CM broth (containing, per of distilled water, 5 g of glucose, 2.5 g of NaCl, 5.0 g of yeast extract, 1.0 g of urea, 10.0 of polypeptone and 5.0 of beef extract, pH 6.8), and the mutagen N-methyl-N'-nitro-N-nitrosoguanidine (NTG) was added thereto to a final concentration of 300 pg/ml, followed by culture with shaking at 200 rpm at 30°C for 20 hours. Thereafter, the culture was centrifuged at 12,000 rpm for 10 minutes to remove the supernatant, and the remaining cells were washed once with saline and further washed three times with phosphate buffer. Then, the cells were suspended in 5 ml of phosphate buffer, plated on a seed culture solid medium (further containing 15 g/1 of agar in addition to the seed culture liquid medium), and cultured at 30°C for 30 hours, and 100 colonies were isolated.

### 1-2. Selection of Mutant Strains Having Enhanced L-lysine Productivity and Construction of Mutant Libraries

Each of the 100 isolated colonies was 5% inoculated into a flask containing 10 ml of the lysine production liquid medium shown in Table 1 below, and was cultured with shaking at 200 rpm at 30°C for 30 hours. Each of the cultures was measured for absorbance at OD 610 nm, and L-lysine production was compared between the cultures. As a result, 10 colonies producing 75.0 g/1 or more of L-lysine were selected. In addition, sequencing was performed to identify the positions of mutations in the promoter of the pyc gene.

**[Table 1]**

| Component | Content (per liter) |
|---|---|
| Glucose | 100 g |
| Ammonium sulfate | 55 g |
| KH₂PO₄ | 1.1 g |
| MgSO₄ · H₂O | 1.2 g |
| MgSO₄ · H₂O | 180 mg |
| FeSO₄ · H₂O | 180 mg |
| Thiamine · HCl | 9 mg |
| Biotin | 1.8 mg |
| CaCO₃ | 5% |
| pH | 7.0 |

### Example 2. Modification of pyc Promoter

### 2-1. Promoter Modification: Introduction of Mutation

30 candidate sequences, each having up to 15 nucleotide modifications, including the positions of mutations in the pyc promoter, which were identified in Example 1, were synthesized by the method described in Sambrook, J. et al. (2001) "Molecular Cloning: A Laboratory Manual", Cold Spring Harbor Laboratory Press, volume 2, 13.36-13.39, and each of the pyc promoter sequence of *Corynebacterium glutamicum* ATCC13032 (see SEQ ID NO: 1) and the synthesized pyc promoter regions was cloned into a pSK1-CAT vector which is a chloramphenicol acetyltransferase (CAT) reporter vector. Orientation during DNA cloning and whether mutation would occur during DNA cloning were examined by DNA sequencing. The mutant libraries constructed as described above were named pSK1-pyc1 to pSK1-pyc30. Finally, each of the mutant libraries was transformed into *Corynebacterium glutamicum* ATCC13032, and the promoter activities were comparatively examined.

### 2-2. Transduction of pSK1-CAT Structure into Corynebacterium glutamicum ATCC13032

In order to transform *Corynebacterium glutamicum* ATCC13032 with each of the constructed pSK1-pyc1 to pSK1-pyc30 identified by sequencing, competent cells were prepared. 10 ml of cultured *Corynebacterium glutamicum* ATCC13032 was inoculated and cultured in 100 ml of BHIS medium at 30°C overnight, and then inoculated into 100 ml of CM broth to reach an OD₆₀₀ of 0.3, and cultured at 18°C at 120 rpm for about 28 hours until it reached an OD₆₀₀ of 0.8. The culture was centrifuged at 6,000 rpm at 4°C for 10 minutes, and the cells were harvested, suspended in 20 ml of 10% glycerol solution, and then centrifuged. This process was repeated three times. The harvested cells were re-suspended in 10% glycerol solution, and 100 µl of the cell suspension was dispensed into each E-tube, and stored in a deep freezer at -70°C until use. 1 µg of DNA was added to 100 µl of the *Corynebacterium glutamicum* ATCC13032 competent cells which were then added to a cooled electroporation cuvette and electroporated using MicroPulser (Bio-Rad). Immediately after pulsing, 1 ml of CM broth pre-warmed at 46°C was added to the cells. Then, the cells were then harvested, kept on ice for 2 minutes, and then incubated in an incubator at 30°C at 180 rpm. Then, 100 µl of the cells were plated on a BHIS-agar plate supplemented with kanamycin (50 pg/ml), and then cultured in an incubator at 30°C.

### 2-3. CAT Assay

Chloramphenicol acetyltransferase (CAT) assay of the pyc promoter region variants was performed using the method of Shaw (Shaw et al., 1991, Biochemistry, 30(44): 10806). Briefly, each of the transformed *Corynebacterium glutamicum* strains was cultured in a CM broth supplemented with kanamycin (50 pg/ml), and the cells were harvested and protein lysates were isolated from the cells. 5 µg of each protein was added to and reacted with 0.1 M Tris-HCl buffer (pH 7.8), 0.4 mg/ml of 5,5'-dithiobis-2-nitrobenzoic acid (DTNB; Sigma D8130), 0.1 mM acetyl CoA (Sigma A2056) and 0.1 mM chloramphenicol at room temperature for 15 minutes, and then the absorbance at OD 412 nm was measured. Thereby, two variants (pSK-pyc3 and pSK-pyc23) showing the greatest increase in CAT activity compared to the *Corynebacterium glutamicum* wild-type pyc promoter sequence were selected.

pSK-pyc3 contained a substitution of tgtggtatgatgg for the nucleotide sequence of the -73 to -61 region and a substitution of acagctgctactgt for the nucleotide sequence of the -51 to -38 region in the promoter sequence upstream of the start codon of the pyruvate carboxylase-encoding gene, and pSK-pyc23 contained a substitution of tgttgtatgattg for the nucleotide sequence of the -73 to -61 region and a substitution of actgctgctactac for the nucleotide sequence of the -51 to -38 region in the promoter sequence. Thereafter, an experiment for verifying the increase in L-lysine productivity by mutation in the promoter of the pyc gene was conducted using the *Corynebacterium glutamicum* DS1 strain.

### Example 3. Construction of Corynebacterium glutamicum Mutant Strain

To construct a *Corynebacterium glutamicum* mutant strain having enhanced activity of pyruvate carboxylase, the *Corynebacterium glutamicum* DS1 strain and *E. coli* DH5a (HIT Competent cells^{™}, Cat No. RH618) were used.

The *Corynebacterium glutamicum* DS1 strain was cultured in a CM-broth medium (pH 6.8) (containing, per liter of distilled water, 5 g of glucose, 2.5 g of NaCl, 5.0 g of yeast extract, 1.0 g of urea, 10.0 g of polypeptone and 5.0 g of beef extract) at a temperature of 30°C.

The *E. coli* DH5a was cultured in an LB medium (containing, per liter of distilled water, 10.0 g of tryptone, 10.0 g of NaCl and 5.0 g of yeast extract) at a temperature of 37°C.

The kanamycin and streptomycin used were purchased from Sigma, and DNA sequencing was performed by Macrogen.

### 3-1. Construction of Recombinant Vector

In order to increase the lysine productivity of the strain by enhancing the supply of the lysine precursor oxaloacetate in the strain, the enhancement of pyruvate carboxylase was introduced. In the method used in this Example, specific mutations in the promoter of the pyc gene were induced in order to increase the expression of the pyc gene encoding pyruvate carboxylase. For substitution of tgtggtatgatgg for the nucleotide sequence ggggttacgatac of the -73 to -61 region and substitution of acagctgctactgt for the nucleotide sequence gtgactgctatcac of the -51 to -38 region in the promoter of the pyc gene, primers including the mutant sequences were constructed. A 735-bp region of the left arm and a 730-bp region of the right arm with respect to the mutated region of the promoter of the pyc gene on the genome of the *Corynebacterium glutamicum* DS1 mutant strain selected in Example 1 were amplified by PCR using the primers. The PCR products were ligated together by overlap PCR, and then cloned into the recombinant vector pCGI (see Kim et al., Journal of Microbiological Methods 84 (2011), 128-130). The resulting plasmid was named pCGI(Pm1-pyc') (see FIG. 1). For construction of the plasmid, the pyc promoter variant-1 amplification primers and pCGI vector amplification primers shown in Table 2 below were used to amplify each DNA fragment.

**[Table 1]**

| Primer (5' → 3') | | | SEQ ID NO |
|---|---|---|---|
| Primers for amplification of pyc promoter variant 1 | pycP-F1 | CATGTATCACGCACTCGGTGAAGGCGTGAGCCC | 4 |
| | pycP-R1 | GACCGCCAAGGACAGTAGCAGCTGTTGCGTCCT ACCATCATACCACACGATTCCC | 5 |
| | pycP-F2 | GGGAATCGTGTGGTATGATGGTAGGACGCAACA GCTGCTACTGTCCTTGGCGGTC | 6 |
| | pycP-R2 | AACTTCTCCAGTGTGATCGCCAAGGATCTGCAC | 7 |
| | pycP-F3 | TGATTACGCCCATGTATCACGCACTCGGTG | 8 |
| | pycP-R3 | GTGTGATCGCCAAGGATCTGCACTTC | 9 |
| Primers for amplification of pCGI vector | pCGI-Fl | ACTGGCCGTCGTTTTACAAC | 10 |
| | pCGI-Rl | GGCGTAATCATGGTCATAGC | 11 |
| | pCGI(pyc)-F2 | TGGAGAAGTTACTGGCCGTCGTTTTACAAC | 12 |
| | pCGI-R2 | TGGTCATAGCTGTTTCCTGTG | 13 |

Specifically, PCR was performed from the genomic DNA of the *Corynebacterium glutamicum* DS1 strain using the corresponding primers under the following conditions.

Using a thermocycler (TP600, TAKARA BIO Inc., Japan) a reaction solution containing 100 µM of each deoxynucleotide triphosphate (dATP, dCTP, dGTP, dTTP), and 1 pM of oligonucleotide, and using 10 ng of the chromosomal DNA of the *Corynebacterium glutamicum* DS1 mutant strain (identified in Example 1) or the pCGI vector as a template, PCR was performed for 25 to 30 cycles in the presence of 1 unit of a pfu-X DNA polymerase mixture (Solgent). The PCR was performed for 25 to 30 cycles, each consisting of (i) denaturation at 94°C for 30 sec, (ii) annealing at 58°C for 30 sec, and (iii) extension at 72°C for 1 to 2 min (a polymerization time of 2 min per kb).

The gene fragments produced as described above were cloned into the pCGI vector by self-assembly cloning. The vector was transformed into *E. coli* DH5a, which was then streaked on an LB-agar plate containing 50 µg/ml of kanamycin and cultured at 37°C for 24 hours. The finally formed colonies were isolated and whether the inserts would be exactly present in the vector was examined, and then the vector was isolated and used for recombination of the *Corynebacterium glutamicum* DS1 strain.

During genetic manipulation, Ex Taq polymerase (Takara) and Pfu polymerase (Solgent) were used as PCR amplification enzymes, and various restriction enzymes and DNA modifying enzymes used were purchased from NEB. These polymerases and enzymes were used according to the supplied buffer and protocols.

### 3-2. Construction of Mutant Strain

A DS5 strain was constructed using the pCGI(Pm1-pyc') vector. The vector was prepared at a final concentration of 1 µg/µl or higher, and introduced into the *Corynebacterium glutamicum* DS1 strain by electroporation (see Tauch et al., FEMS Microbiology letters 123 (1994), 343-347), thus inducing primary recombination. At this time, the electroporated strain was plated on a CM-agar plate containing 20 µg/µl of kanamycin, and the colonies were isolated, and then whether the vector would properly inserted into the induced position on the genome was analyzed by PCR and sequencing. In order to induce secondary recombination of the isolated strain, the isolated strain was inoculated on a CM-agar liquid medium containing streptomycin, cultured overnight or longer, and then plated on an agar medium containing streptomycin at the same concentration, and the colonies were isolated. Whether the final isolated colonies would have resistance to kanamycin was examined, and then whether mutations were introduced into the promoter of the pyc gene in the strains having no antibiotic resistance was analyzed by sequencing (see Schafer et al., Gene 145 (1994), 69-73). Finally, a *Corynebacterium glutamicum* mutant strain (DS5) having the mutations introduced into the promoter of the pyc gene was obtained.

### Example 4. Construction of Corynebacterium glutamicum Mutant Strain

A *Corynebacterium glutamicum* mutant strain was constructed in the same manner as in Example 3, except that substitution of tgttgtatgattg for the nucleotide sequence ggggttacgatac of the -73 to -61 region and substitution of actgctgctactac for the nucleotide sequence gtgactgctatcac of the -51 to -38 region in the promoter of the pyc gene were performed.

In this Example, for construction of a plasmid, the pyc promoter variant-2 amplification primers and pCGI vector amplification primers shown in Table 3 below were used to amplify each DNA fragment, and the constructed plasmid pCGI(Pm2-pyc') vector (see FIG. 2) was used. Finally, a *Corynebacterium glutamicum* mutant strain (DS5-1) having the mutant pyc gene introduced therein was obtained.

**[Table 3]**

| Primer (5' → 3') | | | SEQ ID NO |
|---|---|---|---|
| Primers for amplification of pyc promoter variant 2 | pycP-F1 | CATGTATCACGCACTCGGTGAAGGCGTGAGCC C | 4 |
| | pycP-R4 | GACCGCCAAGGGTAGTAGCAGCAGTTGCGTCC TACAATCATACAACACGATTCCC | 14 |
| | pycP-F4 | GGGAATCGTGTTGTATGATTGTAGGACGCAACT GCTGCTACTACCCTTGGCGGTC | 15 |
| | pycP-R2 | AACTTCTCCAGTGTGATCGCCAAGGATCTGCAC | 7 |
| | pycP-F3 | TGATTACGCCCATGTATCACGCACTCGGTG | 8 |
| | pycP-R3 | GTGTGATCGCCAAGGATCTGCACTTC | 9 |
| Primers for amplification of pCGI vector | pCGI-Fl | ACTGGCCGTCGTTTTACAAC | 10 |
| | pCGI-Rl | GGCGTAATCATGGTCATAGC | 11 |
| | pCGI(pyc)-F2 | TGGAGAAGTTACTGGCCGTCGTTTTACAAC | 12 |
| | pCGI-R2 | TGGTCATAGCTGTTTCCTGTG | 13 |

### Experimental Example 1. Comparison of L-Lysine Productivity between Mutant Strains and Parent Strain

L-lysine productivity was compared between the parent strain *Corynebacterium glutamicum* DS1 strain and the lysine-producing mutant strains DS5 and DS5-1 strains constructed in Examples 3 and 4.

Each of the strains was inoculated into a 100-ml flask containing 10 ml of a lysine medium having the composition shown in Table 1 above, and then cultured with shaking at 180 rpm at 30°C for 28 hours. After completion of the culture, for lysine analysis, the production of L-lysine was measured by HPLC (Shimazu, Japan), and the results of the measurement are shown in Table 4 below.

**[Table 4]**

| Strain name | Positions and sequences of mutations in pyc gene promoter | | L-lysine (g/L) | L-lysine production per gram dry cell weight (g/gDCW) |
|---|---|---|---|---|
| | -73 to -61 | -51 to -38 | | |
| Parent strain (DS1) | ggggttacgatac | gtgactgctatcac | 64.2 | 6.88 |
| Mutant strain (DS5) | tgtggtatgatgg | acagctgctactgt | 69.5 | 8.43 |
| Mutant strain (DS5-1) | tgttgtatgattg | actgctgctactac | 67.2 | 7.51 |

As shown in Table 4 above, it was confirmed that, in the *Corynebacterium glutamicum* mutant strains DS5 and DS5-1 in which specific positions (the -73 to -61 region and the - 51 to -38 region) in the promoter sequence of the pyc gene were substituted with the optimal nucleotide sequences to enhance the supply of the lysine precursor oxaloacetate, the L-lysine productivities of the mutant strains DS5 and DS5-1 increased by about 8.3% and 4.7%, respectively, compared to that of the parent strain *Corynebacterium glutamicum* DS1 strain. From these results, it could be seen that enhanced expression of the pyc gene enhanced L-lysine productivity of the mutant strain by enhancing the supply of the lysine precursor.

As described above, the *Corynebacterium glutamicum* mutant strain according to the present disclosure may produce L-lysine in an improved yield due to increased or enhanced expression of the gene encoding pyruvate carboxylase, compared to the parent strain.

So far, the present disclosure has been described with reference to the embodiments thereof. Those of ordinary skill in the art to which the present disclosure pertains will appreciate that the present disclosure may be embodied in modified forms without departing from the essential

## Claims

1. A *Corynebacterium glutamicum* mutant strain having enhanced L-lysine productivity due to an enhanced activity of pyruvate carboxylase.

2. The *Corynebacterium glutamicum* mutant strain of claim 1, wherein the enhanced activity of the pyruvate carboxylase is achieved by site-directed mutagenesis of a promoter of a gene encoding the pyruvate carboxylase.

3. The *Corynebacterium glutamicum* mutant strain of claim 2, wherein the gene encoding the pyruvate carboxylase is represented by the nucleotide sequence of SEQ ID NO: 1.

4. The *Corynebacterium glutamicum* mutant strain of claim 1, which comprises any one of the nucleotide sequences represented by SEQ ID NOs: 2 and 3.

5. A method for producing L-lysine, the method comprising steps of:
a) culturing the mutant strain of claim 1 in a medium; and
b) recovering L-lysine from the mutant strain or the medium in which the mutant strain has been cultured.

6. Use of a *Corynebacterium glutamicum* mutant strain as defined in any one of claims 1 to 4, for producing L-lysine.
